# FASCICULE DE BREVET EUROPEEN

(11) **EP 0 694 029 B1**
(45) Date de publication et mention de la délivrance du brevet: **29.07.1998**
(21) Numéro de dépôt: 94913646.9
(22) Date de dépôt: 14.04.1994
(51) Int. Cl.: C07C 67/343, C07C 69/734

(54) **NOUVEAU PROCEDE DE PREPARATION D'ACIDE BETA-ALCOXY ACRYLIQUE**
NEUES VERFAHREN ZUR HERSTELLUNG VON BETA-ALKOXYACRYLSÄURE
NOVEL METHOD FOR PREPARING BETA-ALKOXY ACRYLIC ACID

(30) Priorité: 15.04.1993 FR 9304439; 27.01.1994 GB 9401541
(43) Date de publication de la demande: 31.01.1996
(73) Titulaire: ROUSSEL UCLAF, 93230 Romainville (FR)
(72) Inventeur: BRAYER, Jean-Louis, F-60440 Nanteuil-le-Haudouin (FR); HODGSON, David, Michael University of Reading, Reading Barkshire (GB); RICHARDS, Ian, Christopher Chesterford Park, Essex CB10 1XL (GB); WITHERINGTON, Jason University of Reading, Reading Barkshire (GB)
(74) Mandataire: Tonnellier, Marie-José
(86) Numéro de dépôt international: FR9400418
(87) Numéro de publication internationale: WO9424085

(56) Documents cités:
- DATABASE WPI Week 8531, Derwent Publications Ltd., London, GB; AN 85-187547 & JP,A,60 116 650 (UBE INDUSTRIES KK) 24 Juin 1985
- 'CHEMICAL ABSTRACTS SERVICE.REGISTRY HANDBOOK.NUMBER SECTION.1985 SUPPLEMENT' , THE AMERICAN CHEMICAL SOCIETY , COLUMBUS,OHIO,US * RN=99503-30-7 , RN=99503-34-1 * voir page 4264RN
- CHEMICAL ABSTRACTS, vol. 95, no. 23, 7 Décembre 1981, Columbus, Ohio, US; abstract no. 203512c, MIYAURA N ET AL. 'THE PALLADIUM-CATALYZED CROSS-COUPLING REACTION OF PHENYLBORONIC ACIC WITH HALOARENES IN THE PRESENCE OF BASES.' page 640 ;colonne GAUCHE ; & SYNTH.COMM. vol. 11, no. 7 , 1981 pages 513 - 519 MIYAURA N. ET AL
- JOURNAL OF THE CHEMICAL SOCIETY, CHEMICAL COMMUNICATIONS no. 19 , 4 Octobre 1979 , LETCHWORTH GB pages 866 - 867 MIYAURA N ET AL 'STEREOSELECTIVE SYNTHESIS OF ARYLATED (E)-ALCENES BY THE REACTION OF ALK-1-ENYLBORANES WITH ARYL HALIDES IN THE PRESENCE OF PALLADIUM CATALYST'
- TETRAHEDRON LETTERS, 33, no. 31, 1992, R. ROSS et al, pp. 4495-4498

## Description

La présente invention concerne un procédé de préparation de dérivés d'acide β-alkoxy acrylique.

L'invention a pour objet un procédé de préparation de composés de formule (I) : 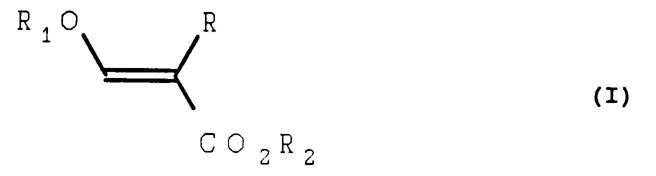 dans laquelle R représente un radical alcényle ou alcynyle renfermant jusqu'à 8 atomes de carbone, éventuellement substitué un radical aryle ou hétéroaryle monocyclique ou polycyclique, éventuellement substitué, R₁ et R₂ identiques ou différents, représentent un radical alkyle renfermant jusqu'à 4 atomes de carbone, caractérisé en ce que l'on soumet un composé de formule (II) : 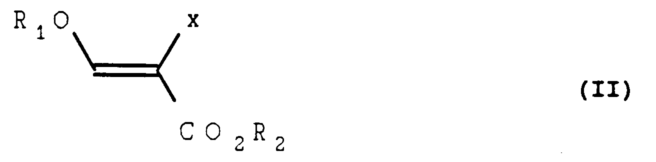 dans laquelle X représente un atome d'halogène, à l'action d'un composé organométallique de formule (III) :

RZ (III)

dans laquelle R conserve sa signification précédente et Z représente un métal ou un dérivé métallique, ou un composé de formule (II') : 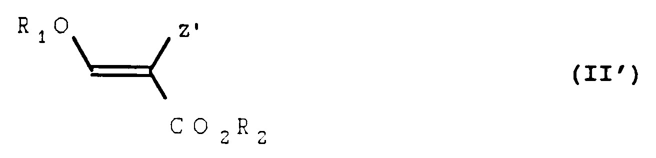 dans laquelle Z' représente un métal ou un dérivé métallique et R₁ et R₂ ont la définition indiquée précédemment, à l'action d'un composé de formule (III') :

RX' (III')

dans laquelle R conserve sa signification précédente et X' représente un groupe nucléofuge, pour obtenir le composé de formule (I) correspondant.

Par radical alkyle que représentent R₁ et R₂, on entend un radical méthyle, éthyle, propyle linéaire ou ramifié, butyle linéaire ou ramifié.

Lorsque R représente un radical alcényle, il s'agit de préférence d'un radical vinyle, allyle, butényle, pentényle, héxényle, ou cyclohéxényle.

Lorsque R représente un radical alcynyle, il s'agit de préférence d'un radical éthynyle, propargyle ou butynyle.

Lorsque R représente un radical aryle, il s'agit de préférence du radical phényle ou naphtyle.

Lorsque le radical alcényle ou alcynyle est substitué, il s'agit par exemple d'un substituant alcoxylé tel que méthoxy, éthoxy, propyloxy.

Lorsque R représente un radical aryle hétérocyclique, il s'agit de préférence d'un radical hétérocyclique à 5 chaînons comme le radical thiényle, furyle, pyrolyle, thiazolyle, oxazolyle, imidazolyle, thiadiazolyle, thiazolidinyle, dioxolannyle, pyrazolyle, oxazolyle ou isoxazolyle, ou encore d'un radical hétérocyclique à 6 chaînons, comme le radical pyridyle, pyrimidinyle, pyridazinyle ou pyrazinyle, ou encore d'un radical aryle condensé comme le radical indolyle benzofuryle, benzothiènyle, benzothiazolyle, quinoléinyle, quinazolinyle ou dihydroquinazolinyle.

Lorsque R est un radical aryle ou hétéroaryle substitué, il est substitué de préférence par un ou plusieurs radicaux choisis dans le groupe constitué par les radicaux carboxyle libre, salifié, estérifié ou amidifié, les radicaux hydroxyle, les atomes d'halogène, les radicaux NH₂, NO₂, C≡N, alkyle, alcényle, alcynyle, aryle et héréroaryle, O-alkyle, O-alcényle, O-alcynyle, O-aryle et O-hétéroaryle, S-alkyle, S-alcényle, S-alcynyle, S-aryle et S-hétéroaryle et N-alkyle, N-alcényle, N-alcynyle, N-aryle et N-hétéroaryle renfermant jusqu'à 24 atomes de carbone, éventuellement substitués par un ou plusieurs atomes d'halogène, le radical 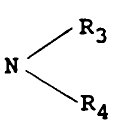 et le radical -S(O)ₜ-R₅,
R₃ et R₄ identiques ou différents, et R₅ représentant un atome d'hydrogène ou un radical alkyle ou aryle renfermant jusqu'à 12 atomes de carbone éventuellement substitué et t représentant le nombre 0, 1 ou 2.

Par atome d'halogène, on entend par exemple un atome de fluor, de chlore, de brome ou d'iode.

Par radical alkyle, alcényle, alcynyle, aryle ou hétéroaryle que peuvent représenter les substituants du radical aryle ou hétéroaryle ou être compris dans ces substituants, on entend de préférence l'un de ceux indiqués ci-dessus pour R₁, R₂ ou R.

Par radical carboxy estérifié, on entend par exemple méthoxycarbonyle ou éthoxycarbonyle.

Par radical carboxy salifié, on entend par exemple un radical salifié par un atome de sodium ou de potassium.

X représente un atome de fluor, de chlore, de brome ou d'iode.

Lorsque Z représente un métal, il s'agit d'un métal alcalin tel que le lithium, le sodium ou le potassium.

Lorsque Z représente un dérivé métallique, il s'agit d'un dérivé de métal alcalino-terreux ou d'un métal de valence supérieure tel que le magnésium, le calcium, le titane, le nickel, le cuivre, le zinc, l'aluminium, le bore, l'étain, le palladium ou le platine.

Par dérivé, on entend un dérivé hydroxylé, alcoxylé, alkylé ou un halogénure, ou encore un dérivé mixte halogéno alkylé ou halogéno hydroxylé.

Par métal ou dérivé métallique que représente Z', on entend l'un de ceux indiqués ci-desus pour Z.

Par groupe nucléofuge que représente X', on entend par exemple un atome d'halogène, de préférence un atome de brome ou d'iode, ou un groupe -O-SO₂-Q dans lequel Q représente un groupement CF₃, (CF₂)ₘ-CF₃ dans lequel m représente un nombre compris entre 2 et 9, un groupement terbutyle ou aryle dans lequel aryle est défini comme ci-dessus.

L'invention a notamment pour objet un procédé caractérisé en ce que dans le composé de formule (II) utilisé comme produit de départ, X représente un atome de brome ou d'iode.

L'invention a particulièrement pour objet un procédé dans lequel Z représente un métal alcalin ou un dérivé de métal alcalino-terreux, de l'étain, du bore ou du zinc.

L'invention a plus particulièrement pour objet, un procédé caractérisé en ce que dans le composé de formule RZ utilisé comme produit de départ, Z est choisi dans le groupe constitué par Li, Na, K, MgHal₁, ZnHal₂, Sn(R')₃, B(OH)₂, B(OR'')₂, B[O(CH₂)ₙO]₂, Hal₁ et Hal₂ représentant un atome d'halogène, R' et R'' représentant un radical alkyle linéaire ou ramifié renfermant jusqu'à 4 atomes de carbone et n représentant un nombre entier égal à 1, 2, 3 ou 4, et tout particulièrement un procédé dans lequel Z est un dérivé du bore comme B(OH)₂, ou encore un procédé dans lequel Z est un dérivé de l'étain comme Sn(R')₃, R' étant défini comme précédemment, par exemple Sn(Bu)₃.

L'invention a également particulièrement pour objet un procédé caractérisé en ce que dans le composé de formule (II'), Z' représente un dérivé de l'étain, du zinc, du cuivre, du palladium ou du platine et de préférence, un dérivé de l'étain Sn(R')₃ défini comme précédemment, par exemple Sn(Bu)₃.

L'invention a encore particulièrement pour objet un procédé caractérisé en ce que le composé de formule (III') est un dérivé halogéné et plus particulièrement un dérivé iodé.

L'invention a tout particulièrement pour objet un procédé caractérisé en ce que la réaction entre les composés de formules (II) et (III) et de formules (II') et (III') a lieu en présence d'un catalyseur au palladium, ou d'un catalyseur au platine ou si désiré en présence d'autres catalyseurs.

Parmi les procédés préférés de l'invention, on peut citer le procédé caractérisé en ce que le catalyseur au palladium est choisi dans le groupe constitué par Pd(OAc)₂, PdCl₂, Pd(Pϕ₃)₄, PdCl₂(CH₃CN)₂, ou encore le tris (dibenzylidène) dipalladium. On peut citer notamment le procédé caractérisé en ce que le catalyseur est Pd(Pϕ₃)₄ ; on peut également citer le procédé caractérisé en ce que la réaction entre les composés de formules (II) et (III) et de formules (II') et (III') a lieu en présence d'un ligand. Le ligand est de préférence une phosphine telle que la triphenylphosphine, une arsine telle que la triphénylarsine ou encore l'acétonitrile ou le benzonitrile. L'invention a tout particulièrement pour objet le procédé caractérisé en ce que le ligand est la triphénylphosphine P(ϕ)₃ ou la triphénylarsine As(ϕ)₃.

Le palladium et le ligand peuvent faire partie d'une même entité chimique, par exemple le cas où l'on utilise Pd(Pϕ₃)₄.

Parmi les procédés de l'invention on peut aussi citer un procédé caractérisé en ce que la réaction entre les composés de formule (II) et de formule (III) a lieu en présence d'une base, par exemple une base choisie dans le groupe constitué par Na₂CO₃, Ba(OH)₂, NaOH, CaCO₃, CsCO₃ et K₃PO₄ et tout particulièrement Na₂CO₃.

Parmi les procédés de l'invention, on peut également citer un procédé caractérisé en ce que la réaction entre les composés de formule (II') et de formule (III') a lieu en présence d'un sel de cuivre, notamment l'iodure cuivreux.

Lorsque l'on soumet un composé de formule (II) dans laquelle X représente un atome de brome ou d'iode, à l'action d'un composé de formule (III) dans laquelle Z représente un dérivé du bore, on peut augmenter de façon considérable la cinétique de couplage en ajoutant un agent de tranfert de phase, par exemple le chlorure de triéthylbenzylammonium.

Les composés de formule (II) utilisés comme produits de départ du procédé de l'invention peuvent être préparés selon le schéma réactionnel suivant : 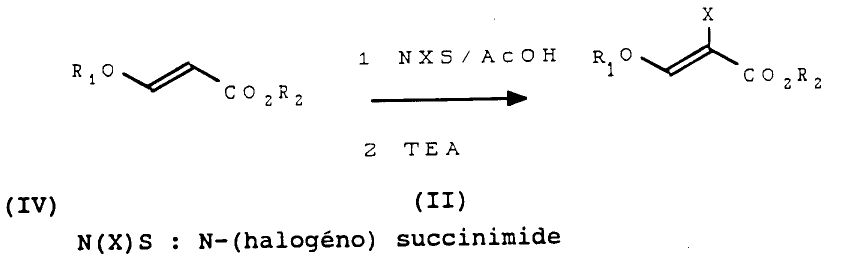 dans lequel X, R₁ et R₂ ont la signification indiquée précédemment.

L'invention met en oeuvre tout particulièrement le 2-iodo 3-méthoxy 2-propénoate de méthyle et le 2-bromo 3-méthoxy 2-propénoate de méthyle, qui est un composé connu décrit dans Database WPi Derwent, week 8531, AN 85-187547 (cf JP-A-60 116 650) et dans C.A.S. Registry Handbook, 1985 suppl., p.4264RN, RN = 99503-30-7 et 99503-34-1.

Les produits de formule (IV) sont des produits connus décrits par exemple dans Tetrahedron letters Vol. 34 n° 47 p. 5209- 5210 (1983).

Les composés de formule (III) sont obtenus de façon classique à partir des dérivés halogénés correspondants selon le procédé décrit par exemple dans Advanced Organic Chemistry 2ème Edition p. 566-569.

Les composés de formule (II') utilisés comme produits de départ du procédé de l'invention ci-dessus décrit sont des produits nouveaux et sont en eux-mêmes un objet de la présente invention : ils peuvent être préparés par couplage organométallique à partir du composé halogéné de formule (II) au sein d'un solvant organique en présence d'un catalyseur au palladium. Un exemple d'une telle préparation est donnée ci-après dans la partie expérimentale.

Les composés de formule (III') sont des produits connus, décrits dans la littérature ou des produits commerciaux.

Le procédé de l'invention est d'un grand intérét industriel, il permet de préparer de façon stéréospécifique en une seule étape, les composés de formule (I), et notamment les dérivés dans lesquels R est un noyau (hétéro) aromatique substitué en position ortho.

Les composés de formule (I) sont des produits connus d'une façon générale décrits par exemple dans le brevet européen 178826.

Les exemples suivants illustrent l'invention sans toutefois la limiter.

### EXEMPLE 1 : (E) α-(méthoxyméthylène) 4-méthylbenzène acétate de méthyle

On porte au reflux pendant 3 heures, 1,44 g de 2-iodo 3-méthoxy 2-propénoate de méthyle obtenu comme indiqué à la préparation 1, 1,22 g d'acide 4-méthyl phénylboronique, 30 ml de toluène, 0,34 g de tétra triphénylphosphine palladium Pd(Pϕ₃)₄ et 6 ml d'une solution de carbonate de sodium 2M. On filtre et rince à l'acétate d'éthyle. On extrait à l'acétate d'éthyle, lave avec une solution de chlorure d'ammonium, sèche les phases organiques, filtre, élimine le solvant sous pression réduite. On obtient 2,06 g de produit que l'on traite au charbon actif, et recristallise dans une solution d'éther isopropylique et de pentane (20-80). On obtient ainsi 1,06 g de produit fondant à 65°C.

### EXEMPLE 2 : (E) α-(méthoxyméthylène) 3-chloro 4-fluoro benzène acétate de méthyle

On porte au reflux pendant une heure et demi un mélange de 1,44 g de 2-iodo 3-méthoxy 2-propénoate de méthyle, 1,57 g d'acide 3-chloro 4-fluorophényl boronique, 30 ml de toluène, 0,07 g d'acétate de palladium, 0,16 g de triphénylphosphine, 2 ml de méthanol et 10 ml d'une solution de carbonate de sodium 2M. On filtre et rince à l'acétate d'éthyle. On extrait à l'acétate d'éthyle et lave avec une solution de chlorure d'ammonium. On sèche les phases organiques et filtre. On concentre sous pression réduite et obtient 2,17 g d'un produit que l'on chromatographie sur silice (éluant : heptane-acétate d'éthyle 85-15). On obtient 1,25 g de produit que l'on traite au charbon actif et recristallise dans l'alcool isopropylique. On obtient ainsi 1,16 g de produit recherché. F = 85,9°C.

### EXEMPLE 3 : (E) α-(méthoxyméthylène) 2-méthylbenzène acétate de méthyle

### STADE A : 2-(2-méthylphényl) 1,3,2-dioxaborinane

On chauffe au reflux pendant 6 heures un mélange de 1,75 g de trimère de l'acide 2-méthyl phénylboronique, 30 ml de cyclohexane, 0,5 ml de propanediol 1,3. On lave à l'eau et extrait au pentane. On sèche les phases organiques et concentre sous pression réduite. On obtient ainsi 1,8 g de produit recherché.

### STADE B : (E) α-(méthoxyméthylène) 2-méthylbenzène acétate de méthyle

On porte à 80°C, pendant une heure un mélange de 0,73 g de 2-iodo 3-méthoxy 2-propanoate de méthyle, 1,02 g de produit préparé au stade A, 20 ml de diméthylformamide, 0,12g de tétra triphénylphosphine palladium Pd(Pϕ₃)₄ et 1,6 g de phosphate tripotassique. On verse le mélange réactionnel sur la glace et extrait à l'éther isopropylique. On extrait, lave, sèche et filtre les phases organiques. On concentre sous pression réduite. On obtient 1,16 g de produit que l'on traite au charbon actif et recristallise dans le pentane. On obtient ainsi 0,54 g de produit recherché, fondant à 43,5°C.

### EXEMPLE 4 : (E) α-(méthoxyméthylène) 2,4-dichlorobenzène acétate de méthyle.

On porte au reflux pendant 4 heures un mélange de 1,44 g de 2-iodo 3-méthoxy 2-propénoate de méthyle, 1,73 g d'acide 2,4-dichlorophényl boronique, 30 ml de toluène, 0,11 g de chlorure de triéthyl benzyl ammonium (TEBAC), 0,30 g de tétrakis triphényl phosphine palladium et 10 ml d'une solution de carbonate de sodium 2M. On filtre, extrait à l'acétate d'éthyle et lave avec une solution de chlorure d'ammonium. On sèche les phases organiques et filtre. On concentre sous pression réduite et obtient 2,17 g d'un produit que l'on chromatographie sur silice (éluant : heptane-acétate d'éthyle 85-15). On obtient le produit brut que l'on recristallise dans une solution d'alcool isopropylique et de pentane 20-80. On obtient 1,31 g de produit recherché. F = 82,1°C.

### EXEMPLE 5 : (E) α-(méthoxyméthylène) 2-méthylbenzène acétate de méthyle.

On porte à 80°C pendant 24 heures, un mélange de 5 ml de 2-(tributylétain) 3-méthoxy 2-propénoate de méthyle obtenu comme à la préparation 3, 1,09 g de 2-iodotoluène, 0,57 g de tétrakis (triphénylphosphine) palladium, 0,71 g d'iodure cuivreux dans 50 ml de diméthylformamide. On filtre, évapore le solvant sous pression réduite, reprend le résidu dans 100 ml d'acétate d'éthyle, ajoute une solution saturée de fluorure de potassium, filtre, lave la phase organique avec une solution aqueuse saturée en chlorure de sodium, sèche et évapore le solvant sous pression réduite. Après chromatographie sur silice (éluant : hexane-acétate d'éthyle 75-25), on récupère 0,57 g de produit brut que l'on recristallise dans le pentane. On obtient 0,5 g de produit attendu. F = 43°C.

Selon le schéma 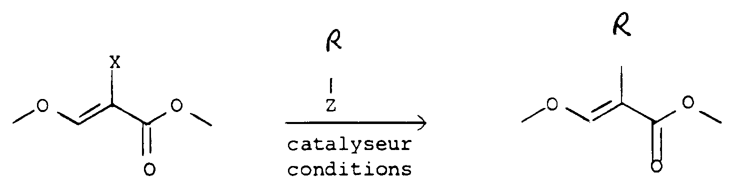 en opérant comme à l'exemple 1, on a obtenu :

| Ex. | R | Z | X | Catalyseur | Conditions | Rendt. % | F°C |
|---|---|---|---|---|---|---|---|
| 6 | phényl | B(OH)2 | I | Pd(PΦ₃)₄ | Na₂CO₃(2H) | 95 | 124,5 |
| 7 | 4-chlorophényl | " | " | " | " | 93 | 63,7 |
| 8 | 3-nitrophényl | " | " | " | " | 53 | 104,4 |
| 9 | 2,4-dichlorophényl | " | " | " | " | 52 | 82,1 |
| 10 | 2,4-diméthylphényl | " | " | " | " | 83 | 67 |
| 11 | naphtyl | " | Br | " | " | 79 | 125 |
| 12 | naphtyl | " | I | " | " | 89 | 125 |
| 13 | vinyl | Sn(Bu)₃ | " | " | - | 76 | - |
| 14 | 1-méthoxyvinyl | " | " | " | - | 82 | - |
| 15 | 2-pyridyl | " | " | " | - | 10 | - |

En opérant comme à l'exemple 3, on a obtenu :

| Ex. | Ar* | Z | X | Catalyseur | Conditions | Rendt.% | F°C |
|---|---|---|---|---|---|---|---|
| 16 | 2-[2,4-dichlorophénoxy] phényl | B(OH)₂ | I | Pd(PΦ₃)₄ | K₃PO₄ | 72 | - |
| 17 | 2-phénoxyphényl | " | " | " | " | 88 | - |

| | | | | | | | |
|---|---|---|---|---|---|---|---|
| * R est Ar. | | | | | | | |

En opérant comme à l'exemple 2, on a obtenu :

| Ex. | Ar* | Z | X | Catalyseur | Conditions | Rendt.% | F°C |
|---|---|---|---|---|---|---|---|
| 18 | 3,5-dichlorophényl | B(OH)₂ | I | Pd(OAc)2 2PΦ₃ | Na₂CO₃(2M) | 79 | 126,5 |
| 19 | 4-fluorophényl | " | " | " | " | 86 | 86,2 |
| 20 | 3,5-bis(trifluorométhyl) phényl | " | " | " | " | 72 | - |
| 21 | 2-méthylphényl | " | " | " | " | 58 | 43,5 |
| 22 | 4-bromophényl | " | " | " | " | 65 | 58,3 |
| 23 | 4-méthoxyphényl | " | " | " | " | 78 | 63 |
| 24 | naphtyl | " | " | " | " | 72 | 125 |
| 25 | 2-phénoxyphényl | " | " | " | " | 52 | - |

| | | | | | | | |
|---|---|---|---|---|---|---|---|
| * R est Ar. | | | | | | | |

En opérant comme à l'exemple 4, on a obtenu :

| Ex. | Ar | Z | X | Catalyseur | Conditions | Rendt.% | F°C |
|---|---|---|---|---|---|---|---|
| 26 | 2-méthylphényl | B(OH)₂ | I | Pd(PΦ₃)₄ | Na₂CO₃(2M)) +TEBAC | 95 | 43,4 |
| 27 | 2-isopropylphényl | | " | " | " | 79 | - |
| 28 | 2-méthyl 3-fluorophényl | | " | " | " | 73 | 65,9 |
| 29 | 2-méthyl 4-fluorophényl | | " | " | " | 80 | 65,5 |
| 29 | 2-méthyl 5-fluoro-phényl | | " | " | " | 75 | 53,2 |

Selon le schéma 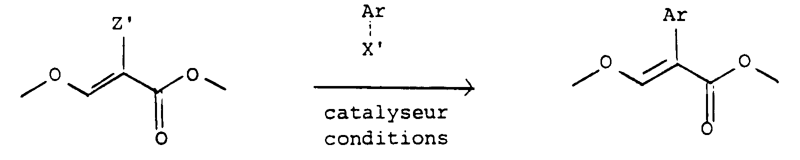 en opérant comme à l'exemple 5, on a obtenu :

| Ex. | Ar | Z' | X' | Catalyseur | Conditions | Rendt.% | F°C |
|---|---|---|---|---|---|---|---|
| 31 | 7-(2,2-diméthyl 3-butynyl) naphtyl | Sn(BU)₃ | I | Pd(PΦ₃)₄ | CuI | 75 | 171,5 |

### EXEMPLE 32 : 3-méthoxy 2-(4-méthylphényl) acrylate de méthyle.

On ajoute sous agitation 63 mg de 4-iodotoluène à une solution comprenant 292 mg de produit obtenu à la préparation 4 et 100 mg de tétrakis (triphénylphosphine) palladium dans 7 cm³ de diméthylformamide. Après 5 minutes, on ajoute 42 mg d'iodure de cuivre et agite le mélange pendant 16 heures à l'abri de la lumière. On ajoute une solution aqueuse de chlorure de sodium puis un mélange 1-1 d'éther de pétrole (Eb. 40°/60°C) et d'éther éthylique. On réunit les phases organiques, sèche et évapore sous pression réduite. On chromatographie le résidu sur silice (éluant : acétate d'éthyleéther de pétrole 4-1) et obtient 43 mg de produit attendu.

### EXEMPLE 33 : 3-méthoxy 2-(2-méthylthiophényl) acrylate de méthyle.

On ajoute sous agitation 41 mg de 1-iodo 2-méthylthiobenzène à une solution comprenant 100 mg de produit obtenu à la préparation 4, 23 mg d'iodure cuivreux, 25 mg de tris (dibenzylidène) dipalladium et 11 mg de triphényl arsine dans 5 ml de N-méthylpyrrolidinone. On chauffe à 50°C pendant 48 heures sous atmosphère inerte et à l'abri de de lumière. On traite le milieu réactionnel comme indiqué à l'exemple 1 et obtient le produit attendu avec un rendement de 79%.

### EXEMPLE 34 : (E) 3-méthoxy 2-(2-phénoxyphényl) acrylate de méthyle.

En opérant comme à l'exemple 33 en utilisant au départ le 1-iodo 2-phénoxybenzène, on a obtenu le produit attendu sous forme d'huile.

### EXEMPLE 35 : (E) 3-méthoxy 2-[(2-phénoxyméthyl) phényl] acrylate de méthyle.

En opérant comme à l'exemple 33 en utilisant au départ le 1-iodo 2-(phénoxyméthyl) benzène, on a obtenu le produit attendu. F = 55°C.

### EXEMPLE 36 : (E) 3-méthoxy 2-[2-(phénylméthylthio) phényl] acrylate de méthyle.

En opérant comme à l'exemple 33 en utilisant au départ le 1-iodo 2-(phénylméthylthio) benzène, on a obtenu le produit attendu. F = 72-74°C.

### EXEMPLE 37 : (E) 3-méthoxy 2-[3-méthyl 2-(phénylméthylthio) phényl] acrylate de méthyle.

En opérant comme à l'exemple 33 en utilisant au départ le 1-iodo 3-méthyl 2-(phénylméthylthio) benzène, on a obtenu le produit attendu. F = 119-120°C.

### EXEMPLE 38 : 3-méthoxy 2-(2-thiophényl) acrylate de méthyle.

En opérant comme à l'exemple 33 en utilisant au départ le 2-iodo thiophène, on a obtenu le produit attendu sous forme d'huile comprenant un mélange 2/1 d'isomères E et Z que l'on purifie par chromatographie sur silice.

### Préparation 1 : 2-iodo 3-méthoxy 2-propénoate de méthyle

On introduit une solution renfermant 12,95 g de 3-méthoxy 2-propénoate de méthyle et 50 ml d'acide acétique dans une solution renfermant 37 g de N-iodosuccinimide et 150 ml d'acide acétique. On agite pendant 4 heures à la température ambiante. On concentre sous pression réduite et reprend à l'éther isopropylique. On filtre et rince à l'éther isopropylique. On concentre, reprend au chlorure de méthylène et additionne une quantité suffisante de triéthylamine pour assurer la conversion en dérivé acrylique. On lave, sèche et concentre. On obtient une huile qui cristallise spontanément à la température ambiante. On recristallise les cristaux obtenus dans l'isopropanol. On obtient 17 g de produit recherché fondant à 50,9°C.

### Préparation 2 : 2-bromo 3-méthoxy 2-propénoate de méthyle

On maintient au reflux pendant 2 heures, un mélange de 17 g de 3-méthoxy 2-propénoate de méthyle, 200 ml d'acétonitrile et 26,1 g de N-bromo succinimide.

On ajoute 2 g de N-bromosuccinimide et porte à nouveau au reflux pendant 2 heures.

On concentre sous pression réduite, et reprend à l'aide d'une solution aqueuse de bicarbonate de sodium. On extrait à l'éther isopropylique, lave, sèche et concentre. On obtient une huile qui est distillée sous pression réduite. On obtient ainsi 15 g de produit qui cristallise spontanément à la température ambiante.

On recristallise dans le pentane les cristaux obtenus. On obtient ainsi le produit recherché. F = 30,1°C.

| Microanalyse : | | |
|---|---|---|
| | Théorique | Trouvé |
| C | 30,79 | 30,8 |
| H | 3,62 | 3,6 |
| Br | 40,98 | 40,8. |

### Preparation 3 : 2-(tributylétain) 3-méthoxy 2-propénoate de méthyle.

On chauffe 12 heures au reflux, 10 g de 2-iodo 3-méthoxy 2-propénoate de méthyle obtenu comme à la préparation 1 et 50 ml de hexabutyldiétain en présence de 1 g de chlorure de bis (triphényl phosphine) palladium dans 300 ml de dioxanne. On élimine le solvant sous pression réduite, reprend le résidu dans 300 ml d'acétate d'éthyle, ajoute une solution saturée de fluorure de potassium, filtre, lave la phase organique avec une solution aqueuse saturée en chlorure de sodium, sèche et évapore le solvant sous pression réduite. Après chromatographie sur silice (éluant : hexane-acétate d'éthyle 98-2), on obtient 9,5 g de produit attendu.

### Préparation 4 : E-3-méthoxy 2-tri-n-butylstannyl propenoate de méthyle.

On ajoute sous agitation à température ambiante 100 mg de tétrakis (triphénylphosphine) palladium (O) à une solution comprenant 750 mg de 2-bromo 3-méthoxy propenoate de méthyle et 3,87 ml de bis (tri-n-butyline) dans 5 ml de toluène. On chauffe le mélange au reflux et agite pendant 24 heures. On refroidit, ajoute 30 ml d'éther diéthylique et 5 ml de solution aqueuse saturée en fluorure de potassium et agite 15 minutes ; on filtre sur celite, lave avec une solution aqueuse de chlorure de sodium, sèche sur sulfate de magnésium et évapore sous pression réduite. On purifie par chromatographie sur silice (éther de pétrole-acétate d'éthyle 95-5) et obtient le produit attendu.

En opérant de manière identique à celle décrite à la préparation 4, en utilisant au départ le 2-iodo 3-méthoxy propenoate de méthyle, on a préparé le E-3-méthoxy 2-tri-n-butylstannyl propenoate de méthyle.

## Revendications

1. Procédé de préparation de composés de formule (I) : 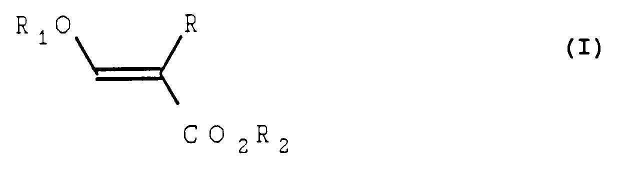 dans laquelle R représente un radical alcényle ou alcynyle renfermant jusqu'à 8 atomes de carbone éventuellement substitué, un radical aryle ou hétéroaryle monocyclique ou polycyclique, éventuellement substitué, R₁ et R₂ identiques ou différents représentent un radical alkyle renfermant jusqu'à 4 atomes de carbone caractérisé en ce que l'on soumet un composé de formule (II) : 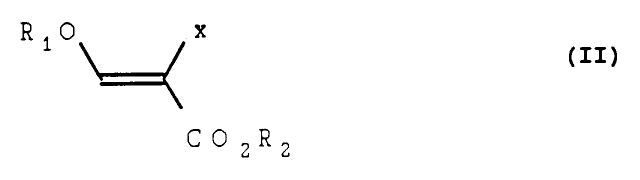 dans laquelle X représente un atome d'halogène, à l'action d'un composé organométallique de formule (III) :
RZ (III)
dans laquelle R conserve sa signification précédente et Z représente un métal ou un dérivé métallique, ou un composé de formule (II') : 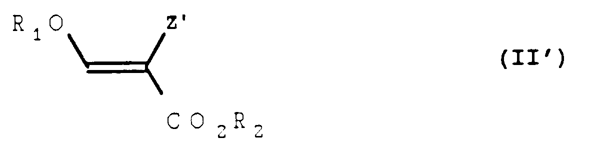 dans laquelle Z' représente un métal ou un dérivé métallique et R₁ et R₂ ont la définition indiquée ci-dessus, à l'action d'un composé de formule (III') :
RX' (III')
dans laquelle R conserve la signification indiquée ci-dessus et X' représente un groupe nucléofuge, pour obtenir le composé de formule (I) correspondant.

2. Procédé selon la revendication 1, caractérisé en ce que X représente un atome de brome ou d'iode.

3. Procédé selon la revendication 1 ou 2, caractérisé en ce que Z représente un métal alcalin ou un dérivé de métal alcalino-terreux, de l'étain, du bore ou du zinc.

4. Procédé selon l'une quelconque des revendications 1 à 3, caractérisé en ce que Z est choisi dans le groupe constitué par Li, Na, K, MgHal₁, ZnHal₂, Sn(R')₃, B(OH)₂, B(OR'')₂, B[O(CH₂)ₙO]₂, Hal₁ et Hal₂ représentant un atome d'halogène, R' et R'' représentant un radical alkyle linéaire ou ramifié renfermant jusqu'à 4 atomes de carbone et n représentant un nombre entier égal à 1, 2, 3 ou 4.

5. Procédé selon la revendication 4, caractérisé en ce que Z est un dérivé du bore.

6. Procédé selon la revendication 5, caractérisé en ce que le dérivé du bore est B(OH)₂.

7. Procédé selon la revendication 4, caractérisé en ce que Z est un dérivé de l'étain Sn(R')₃, R' étant défini comme à la revendication 4.

8. Procédé selon la revendication 7, caractérisé en ce que le dérivé de l'étain est Sn(Bu)₃.

9. Procédé selon la revendication 1, caractérisé en ce que dans le composé de formule (II'), Z' représente un dérivé de l'étain, du zinc, du cuivre, du palladium ou du platine.

10. Procédé selon la revendication 9, caractérisé en ce que Z' est un dérivé de l'étain.

11. Procédé selon la revendication 9, caractérisé en ce que le dérivé de l'étain est Sn(Bu)₃.

12. Procédé selon la revendication 9, caractérisé en ce que le composé de formule (III') est un dérivé halogéné.

13. Procédé selon la revendication 12, caractérisé en ce que le dérivé halogéné est un dérivé iodé.

14. Procédé selon l'une quelconque des revendications 1 à 13, caractérisé en ce que la réaction entre les composés de formules (II) et (III) ou de formules (II') et (III') a lieu en présence d'un catalyseur au palladium, ou d'un catalyseur au platine.

15. Procédé selon l'une quelconque des revendications 1 à 14, caractérisé en ce que le catalyseur au palladium est choisi dans le groupe constitué par Pd(OAc)₂, PdCl₂, Pd(Pϕ₃)₄, PdCl₂(CH₃CN)₂ ou le tris(dibenzylidène) dipalladium.

16. Procédé selon la revendication 15, caractérisé en ce que le catalyseur est Pd(Pϕ₃)₄.

17. Procédé selon l'une quelconque des revendications 1 à 16, caractérisé en ce que la réaction entre les composés de formules (II) et (III) ou de formules (II') et (III') a lieu en présence d'un ligand.

18. Procédé selon la revendication 17, caractérisé en ce que le ligand est une phosphine, une arsine, l'acétonitrile ou le benzonitrile.

19. Procédé selon la revendication 18, caractérisé en ce que le ligand est choisi dans le groupe constitué par P(ϕ)₃ et As(ϕ)₃.

20. Procédé selon l'une quelconque des revendications 1 à 8 et 14 à 19, caractérisé en ce que la réaction entre les composés de formule (II) et de formule (III) a lieu en présence d'une base.

21. Procédé selon la revendication 20, caractérisé en ce que la base est choisie dans le groupe constitué par Na₂CO₃, Ba(OH)₂, NaOH, CaCO₃, CsCO₃ et K₃PO₄.

22. Procédé selon la revendication 21, caractérisé en ce que la base est Na₂CO₃.

23. Procédé selon l'une quelconque des revendications 1 et 9 à 19, caractérisé en ce que la réaction entre les composés de formule (II') et de formule (III') a lieu en présence d'un sel de cuivre.

24. Procédé selon la revendication 23, caractérisé en ce que le sel de cuivre est l'iodure cuivreux.

## Claims

1. Preparation process for the compounds of formula (I): 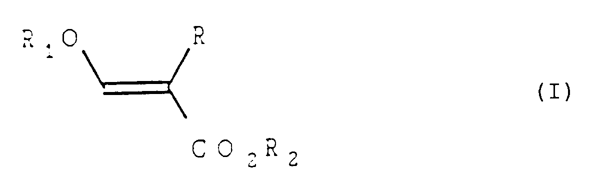 in which R represents an optionally substituted alkenyl or alkynyl radical containing up to 8 carbon atoms, an optionally substituted aryl or a monocyclic or polycyclic heteroaryl radical, R₁ and R₂, identical or different, represent an alkyl radical containing up to 4 carbon atoms, characterized in that a compound of formula (II): 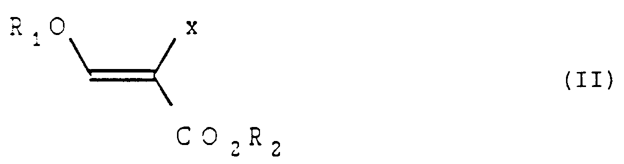 in which X represents a halogen atom, is subjected to the action of an organometallic compound of formula (III):
RZ (III)
in which R retains its previous meaning and Z represents a metal or a metallic derivative, or a compound of formula (II'): 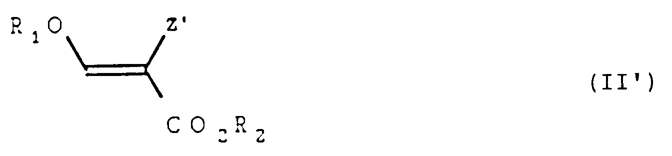 in which Z ' represents a metal or a metallic derivative and R₁ and R₂ have the meaning indicated above, is subjected to the action of a compound of formula (III'):
RX' (III')
in which R retains the meaning indicated above and X' represents a nucleofugal group, in order to obtain the corresponding compound of formula (I).

2. Process according to claim 1, characterized in that X represents a bromine or iodine atom.

3. Process according to claim 1 or 2, characterized in that Z represents an alkali metal or a derivative of an alkaline-earth metal, of tin, boron or zinc.

4. Process according to any one of claims 1 to 3, characterized in that Z is chosen from the group constituted by Li, Na, K, MgHal₁, ZnHal₂, Sn(R')₃, B(OH)₂, B(OR'')₂, B[O(CH₂)ₙO]₂, Hal₁ and Hal₂ representing a halogen atom, R' and R'' representing a linear or branched alkyl radical containing up to 4 carbon atoms and n representing an integer equal to 1, 2, 3 or 4.

5. Process according to claim 4, characterized in that Z is a derivative of boron.

6. Process according to claim 5, characterized in that the derivative of boron is B(OH)₂.

7. Process according to claim 4, characterized in that Z is a derivative of tin Sn(R')₃, R' being as defined in claim 4.

8. Process according to claim 7, characterized in that the derivative of tin is Sn(Bu)₃.

9. Process according to claim 1, characterized in that in the compound of formula (II'), Z' represents a derivative of tin, zinc, copper, palladium or platinum.

10. Process according to claim 9, characterized in that Z' is a derivative of tin.

11. Process according to claim 9, characterized in that the derivative of tin is Sn(Bu)₃.

12. Process according to claim 9, characterized in that the compound of formula (III') is a halogenated derivative.

13. Process according to claim 12, characterized in that the halogenated derivative is an iodated derivative.

14. Process according to any one of claims 1 to 13, characterized in that the reaction between the compounds of formulae (II) and (III) or formulae (II') and (III') takes place in the presence of a palladium catalyst, or a platinum catalyst.

15. Process according to any one of claims 1 to 14, characterized in that the palladium catalyst is chosen from the group constituted by Pd(OAc)₂, PdCl₂, Pd(Pφ₃)₄, PdCl₂(CH₃CN)₂ or tris(dibenzylidene) dipalladium.

16. Process according to claim 15, characterized in that the catalyst is Pd(Pφ₃)₄.

17. Process according to any one of claims 1 to 16, characterized in that the reaction between the compounds of formulae (II) and (III) or formulae (II') and (III') takes place in the presence of a ligand.

18. Process according to claim 17, characterized in that the ligand is a phosphine, an arsine, acetonitrile or benzonitrile.

19. Process according to claim 18, characterized in that the ligand is chosen from the group constituted by P(φ)₃ and As(φ₃).

20. Process according to any one of claims 1 to 8 and 14 to 19, characterized in that the reaction between the compounds of formula (II) and formula (III) takes place in the presence of a base.

21. Process according to claim 20, characterized in that the base is chosen from the group constituted by Na₂CO₃, Ba(OH)₂, NaOH, CaCO₃, CsCO₃ and K₃PO₄.

22. Process according to claim 21, characterized in that the base is Na₂CO₃.

23. Process according to any one of claims 1 and 9 to 19, characterized in that the reaction between the compounds of formula (II') and formula (III') takes place in the presence of a copper salt.

24. Process according to claim 23, characterized in that the copper salt is cuprous iodide.

## Patentansprüche

1. Verfahren zur Herstellung von Verbindungen der Formel (I) 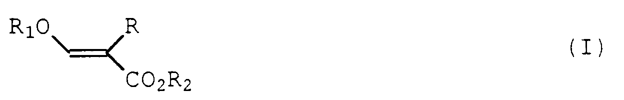 in der
R einen Rest Alkenyl oder Alkinyl mit bis zu 8 Kohlenstoffatomen, gegebenenfalls substituiert, einen monocyclischen oder polycyclischen Rest Aryl oder Heteroaryl, gegebenenfalls substituiert, darstellt, R₁ und R₂, gleich oder verschieden, einen Rest Alkyl mit bis zu 4 Kohlenstoffatomen bedeuten, dadurch gekennzeichnet, daß man eine Verbindung der Formel (II) 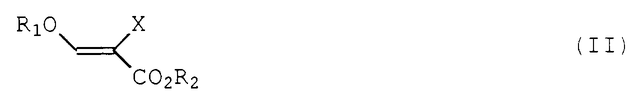 in der X ein Halogenatom ist, der Einwirkung einer Organometallverbindung der Formel (III)
RZ (III)
unterzieht, in der R die oben angegebene Bedeutung behält und Z ein Metall oder ein Metallderivat bedeutet, oder eine Verbindung der Formel (II') 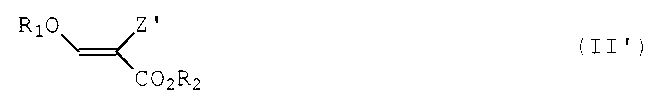 in der Z' ein Metall oder ein Metallderivat darstellt und R₁ und R₂ die oben angegebene Bedeutung besitzen, der Einwirkung einer Verbindung der Formel (III')
RX' (III')
unterzieht, in der R die oben angegebene Bedeutung behält und X' eine kernflüchtige Gruppe ist, um die entsprechende Verbindung der Formel (I) zu erhalten.

2. Verfahren nach Anspruch 1, dadurch gekennzeichnet, daß X ein Bromatom oder Iodatom darstellt.

3. Verfahren nach Anspruch 1 oder 2, dadurch gekennzeichnet, daß Z ein Alkalimetall oder ein Derivat von einem Erdalkalimetall, von Zinn, Bor oder Zink darstellt.

4. Verfahren nach irgendeinem der Ansprüche 1 bis 3, dadurch gekennzeichnet, daß Z aus der Gruppe gewählt wird, die gebildet wird durch Li, Na, K, MgHal₁, ZnHal₂, Sn(R')₃, B(OH)₂, B(OR'')₂, B[O(CH₂)ₙO]₂, worin Hal₁ und Hal₂ ein Halogenatom darstellen, R' und R'' einen geraden oder verzweigten Rest Alkyl mit bis zu 4 Kohlenstoffatomen bedeuten und n eine ganze Zahl von 1, 2, 3 oder 4 ist.

5. Verfahren nach Anspruch 4, dadurch gekennzeichnet, daß Z ein Borderivat ist.

6. Verfahren nach Anspruch 5, dadurch gekennzeichnet, daß das Borderivat B(OH)₂ ist.

7. Verfahren nach Anspruch 4, dadurch gekennzeichnet, daß Z ein Zinnderivat Sn(R')₃ ist, worin R' wie in Anspruch 4 definiert ist.

8. Verfahren nach Anspruch 7, dadurch gekennzeichnet, daß das Zinnderivat Sn(Bu)₃ ist.

9. Verfahren nach Anspruch 1, dadurch gekennzeichnet, daß in der Verbindung der Formel (II') Z' ein Derivat von Zinn, Zink, Kupfer, Palladium oder Platin ist.

10. Verfahren nach Anspruch 9, dadurch gekennzeichnet, daß Z' ein Derivat von Zinn ist.

11. Verfahren nach Anspruch 9, dadurch gekennzeichnet, daß das Zinnderivat Sn(Bu)₃ ist.

12. Verfahren nach Anspruch 9, dadurch gekennzeichnet, daß die Verbindung der Formel (III') ein halogeniertes Derivat ist.

13. Verfahren nach Anspruch 12, dadurch gekennzeichnet, daß das halogenierte Derivat ein iodiertes Derivat ist.

14. Verfahren nach irgendeinem der Ansprüche 1 bis 13, dadurch gekennzeichnet, daß die Reaktion zwischen den Verbindungen der Formeln (II) und (III) oder den Verbindungen der Formeln (II') und (III') in Anwesenheit eines Palladiumkatalysators oder eines Platinkatalysators stattfindet.

15. Verfahren nach irgendeinem der Ansprüche 1 bis 14, dadurch gekennzeichnet, daß der Palladiumkatalysator aus der Gruppe gewählt wird, die gebildet wird durch Pd(OAc)₂, PdCl₂, Pd(Pϕ₃)₄, PdCl₂(CH₃CN)₂ oder Tris-(Dibenzyliden)-dipalladium.

16. Verfahren nach Anspruch 15, dadurch gekennzeichnet, daß der Katalysator Pd(Pϕ₃)₄ ist.

17. Verfahren nach irgendeinem der Ansprüche 1 bis 16, dadurch gekennzeichnet, daß die Reaktion zwischen den Verbindungen der Formeln (II) und (III) oder den Verbindungen der Formeln (II') und (III') in Anwesenheit eines Liganden stattfindet.

18. Verfahren nach Anspruch 17, dadurch gekennzeichnet, daß der Ligand ein Phosphin, ein Arsin, Acetonitril oder Benzonitril ist.

19. Verfahren nach Anspruch 18, dadurch gekennzeichnet, daß der Ligand aus der Gruppe gewählt wird, die gebildet wird durch P(ϕ₃) und As(ϕ₃).

20. Verfahren nach irgendeinem der Ansprüche 1 bis 8 und 14 bis 19, dadurch gekennzeichnet, daß die Reaktion zwischen den Verbindungen der Formeln (II) und (III) in Anwesenheit einer Base stattfindet.

21. Verfahren nach Anspruch 20, dadurch gekennzeichnet, daß die Base aus der Gruppe gewählt wird, die gebildet wird durch Na₂CO₃, Ba(OH)₂, NaOH, CaCO₃, CsCO₃ und K₃PO₄.

22. Verfahren nach Anspruch 21, dadurch gekennzeichnet, daß die Base Na₂CO₃ ist.

23. Verfahren nach irgendeinem der Ansprüche 1 und 9 bis 19, dadurch gekennzeichnet, daß die Reaktion zwischen den Verbindungen der Formeln (II') und (III') in Anwesenheit eines Kupfersalzes stattfindet.

24. Verfahren nach Anspruch 23, dadurch gekennzeichnet, daß das Kupfersalz Kupfer(I)-iodid ist.
